# EUROPÄISCHE PATENTSCHRIFT

(11) **EP 2 077 124 B1**
(45) Veröffentlichungstag und Bekanntmachung des Hinweises auf die Patenterteilung: **23.04.2014**
(21) Anmeldenummer: 08000038.3
(22) Anmeldetag: 03.01.2008
(51) Int. Cl.: A61L 27/04, A61L 27/30, A61L 27/50

(54) **Biokompatible Oberflächenschicht**
Bio-compatible surface layer
Couche superficielle biocompatible

(43) Veröffentlichungstag der Anmeldung: 08.07.2009
(73) Patentinhaber: ZL Microdent-Attachment GmbH & Co. KG, 58339 Breckerfeld (DE)
(72) Erfinder: Böschemeyer, Thomas, 58313 Herdecke (DE); Clostermann, Volkhard-Hagen, 58097 Hagen (DE); Graf, Hans-Ludwig, Prof. Dr., 04451 Borsdorf (DE); Schreckenbach, Joachim P., Dr., 09337 Hohenstein-Ernstthal (DE)
(74) Vertreter: Kötter, Ulrich

(56) Entgegenhaltungen:
- WO-A-2004/024202
- WO-A-2006/043168
- US-A1- 2002 198 601

## Beschreibung

Die Erfindung betrifft einen Körper mit biokompatibler Oberflächenschicht nach dem Oberbegriff des Patentanspruchs 1. Die Erfindung betrifft darüber hinaus ein Verfahren zur Beschichtung eines Substrats nach dem Patentanspruch 14.

Der Einsatz zahnmedizinischer Implantate ist fester Bestandteil der Zahnmedizin. Derartige Implantate sind in den unterschiedlichsten Ausführungen bekannt und werden entweder in künstlich in einen Kiefer eingebrachte Öffnungen eingeschraubt oder - sofern der Knochenkontaktbereich eines Implantats der Wurzelform eines extrahierten Zahnes nachgebildet wurde - in das so entstandene, leere Zahnfach eingesetzt. Nach vollständiger Einheilung des Implantats wird in dieses ein Aufbau eingeschraubt, an dem eine Zahnkrone befestigt ist. Auf Grund seiner biokompatiblen Eigenschaften ist das Implantat sowie der Aufbau dabei regelmäßig aus Titan hergestellt. Die an dem Aufbau befestigte Zahnkrone ist in der Regel aus Zirkoniumoxid / -dioxid hergestellt. Die Farbe des Zirkoniumoxids / -dioxids und die biotechnischen Charakteristika dieses Materials ermöglichen qualitativ hochwertige und ästhetische Zahnrekonstruktionen.

Nachteilig an den bekannten Implantatsystemen ist, dass im Falle eine Zahnfleisch- und / oder Knochenrückgangs das titanfarbene Implantat zum Vorschein kommt, welches metallfarben schimmert und vielfach als ästhetisch störend empfunden wird. Gesucht ist an dieser Stelle eine geeignete Möglichkeit, das titanfarbene Implantat mit einer biokompatiblen Schicht aus Zirkoniumoxid / -dioxid zu versehen.

Es ist bekannt, den Halsbereich eines Implantats zu verjüngen und mit einer Hülse aus Zirkoniumoxid / -dioxid zu versehen, welche mittels bekannter Fügeverfahren an dem Implantat befestigt wird. Diese Lösung erweist sich als sehr aufwendig. Darüber hinaus besteht die Gefahr, dass sich die Keramikhülse im Laufe der Zeit lockert.

Aus der Patentschrift DD 210 607 ist die Beschichtung von Metallen durch Oxidschichten bekannt. Beispielsweise wird hier ein mit Aluminium überzogenes Edelstahlimplantat mit einer anodischen Oxidschicht versehen. Derartige Schichten weisen jedoch den Nachteil auf, dass diese nur eine geringe Biokompatibilität aufweisen.

Zu den bereits etablierten Verfahren zum Auftragen von Oxiden auf Oberflächen zählt das Plasma- oder Elektro-Spraying, wie es beispielsweise die
EP 157 54 42 A1 offenbart. Dabei werden entsprechend vorbereitete oxidkeramische Versätze unter verfahrensspezifischen Parametern auf Oberflächen aufgespritzt. Neben dem hohen technischen Verfahrensaufwand erweist sich hier die Beschichtung komplexer Geometrien, wie Hinterschneidungen und Hohlräume als problematisch.

In der WO 03/039609 A1 wird die elektrophoretische Beschichtung von Implantaten mit Hydroxylapatit als bioaktives Oberflächenmaterial vorgeschlagen. Derartige Schichten erweisen sich jedoch wegen ihrer geringen Haftfestigkeit als instabil.

In der WO 92/13954, WO 00/7277 sowie WO 00/72776 sind haftfeste Schichten auf der Basis von Titanoxid auf Titansubstraten beschrieben, welche durch anodische Oxidation in phosphathaltigen Elektrolysen erzielt werden. In der WO 02/078759 ist vorgeschlagen, bioaktive Oberflächenschichten auf Titanimplantaten mittels Ethylendiamintetraessigsäure-haltigen Elektrolyten zu erzielen. Alle diese vorgenannten Lösungen beschränken sich jedoch auf den Verbund von Titanoxidschichten (teils Ca-Phosphat-haltig) auf Titansubstraten. Darüber hinaus ist beim Einsatz organischer Verbindungen eine mögliche und ungewollte chemische Veränderung dieser Verbindungen während der Herstellung oder Lagerung der Implantate möglich.

In der WO 03/045268 A1 werden ein keramisches Dentalimplantat sowie ein Herstellungsverfahren für dieses offenbart. Hierbei handelt es sich um ein einstückiges Vollkeramikimplantat aus einem Werkstoff auf Zirkoniumoxidbasis. Um die Problematik der Bioinertheit von hochverdichteter Zirkoniumkeramik zu reduzieren, wird die Oberfläche des Verankerungsteils dieses Implantats aufgerauht. Hierzu werden Strahlbehandlungen (Korund-Borcarbid), Hochdruckwasserstrahlen, lasergestützte abtragende Verfahren oder chemische Ätzverfahren vorgeschlagen. Nachteilig hierbei ist, dass es bei allen diesen nachträglich eingesetzten Verfahren zur Ausbildung von potenziellen Oberflächenschädigungen kommen kann, welche Ausgangspunkte für Mikrorisse sind, die wiederum zu regelmäßig beobachtbaren Totalbrüchen solcher Implantate führen können.

Unter Ausnutzung der vorteilhaften biologischen Eigenschaften von Zirkoniumoxid und der mechanischen Stabilität von Titan wird in der WO 2006/043168 A2 die Beschichtung von Titan-Körpern beschrieben. Hierbei werden gelöste Zirkoniumalkoxykomplexe auf die Implantatoberfläche aufgebracht (sogenanntes Dip-Coating) und anschließend thermischen Nachbehandlungsregimen unterzogen, bis dünne Zirkoniumoxidfilme an der Oberfläche entstehen. Derartige Oberflächen weisen jedoch eine nur geringe Haftung zum Basismetall auf.

Einen vergleichbaren Ansatz offenbart EP 1 566 152 A1. Hier wird eine Zirkoniumoxidschicht auf Titan aufgesintert. Nachteilig ist hier besonders, dass bei den hohen Sintertemperaturen die für die mechanische Stabilität des Titans verantwortlichen metallischen Gefügestrukturen des Grundmetalls verändert werden.

In der DE 263 29 32 A1 und DE 195 06 188 A1 sind Implantatoberflächen beschrieben, die mittels Verfahren aus der Gruppe der CVD-(chemical vapour deposition) oder PVD-(physical vapour depositon) Techniken auf Implantatkörper aufgetragen werden. Derartige, aus Nitriden oder Oxiden bestehende Oberflächen besitzen jedoch auf Grund ihrer im nm-Bereich liegenden Schichtdicken keine biologisch attraktive Oberflächenstrukturierung. Die Bildung von Zirkoniumoxiden durch rein thermische Oxidation von Zirkonium oder Zirkoniumlegierungen beschreibt die US 644 75 50 B1. Hier wird eine porenfreie, glatte Oxidschicht (Zunderschicht) auf dem metallischen vorstrukturierten Zirkoniumkörper ausgebildet.

In der WO 2004/024202 ist die anodische Behandlung in phosphathaltigen Elektrolyten beschrieben, wodurch sehr dünne, unstrukturierte Oberflächenoxidschichten erhalten werden. Es entsteht jeweils das zum Substrat korrespondierende Oxid (TiO₂ auf Ti, ZrO₂ auf Ti, ZrO₂ auf Zr). Eine mögliche Porosität der Oberfläche wird durch die Vorstrukturierung des metallischen Substrats festgelegt.

Zu einer möglichen Lösung des Problems der Erzeugung poröser, bioaktiver Zirkoniumoxidoberflächen wurde eine Technologie entwickelt, bei der auf einem vorgesinterten keramischen Zirkonoxidrohling durch nachträgliches Auftragen geeigneter Suspensionen weitere keramische, zirkonoxidhaltige Schichten, welche zusätzlich Spacer in Form von beispielsweise Grafit- oder Kunststoffpartikeln enthalten, aufgebracht werden (WO 2005/027771 A1). Bei dem sich anschließenden Brennvorgang werden diese Spacer ausgebrannt und hinterlassen entsprechende poröse Strukturen.

Ein generelles Problem bei allen keramischen Brennvorgängen von Zirkoniumoxid ist dessen Umwandlung in tetragonale oder kubische Oxidphasen bei Temperaturen ab etwa 1000° Celsius. Daher werden diesen keramischen Massen beispielsweise Yttriumoxide zugesetzt, um die tetragonale Hochtemperaturphase bei der späteren Abkühlung auf Raumtemperatur zu stabilisieren. Eine unkontrollierte Umwandlung tetragonaler Zirkoniumoxidmodifikation zur monoklinen Tieftemperaturphase führt in Folge der starken Volumenzunahme des Zirkonoxids zum mechanischen Bruch bzw. zum Zerfall solcher Keramikkörper. Aber auch stabilisiertes tetragonales Zirkonoxid neigt, insbesondere unter Feuchtigkeit und mechanischer Beanspruchung, zur Alterung, das heißt, es wird die monokline Phase gebildet und die ursprüngliche mechanische Stabilität der Keramik geht verloren. Wegen problematischer mechanischer Eigenschaften wird daher der Einsatz von vollkeramischen, Zirkonium (IV)-Oxid basierenden Materialien in der Literatur als kritisch bewertet (J. Chevalier, Biomaterials 27 (2006) 535).

Der bestehende Zielkonflikt zwischen hoher mechanischer Stabilität bei porenfreien Zirkonoxiden mit unstrukturierter Oberfläche einerseits und porösen, biokompatiblen aber mechanisch wenig stabilen Zirkonoxiden andererseits ist zu lösen.

Der Erfindung liegt daher die Aufgabe zu Grunde, die bekannten Nachteile des Standes der Technik zu vermeiden und eine verbesserte Oberflächenschicht, insbesondere für medizinische Implantate und Prothesen zu schaffen, bei welcher die besonderen Vorteile von Zirkonoxiden für biologische Anwendungen genutzt werden und bei der gleichzeitig eine hohe mechanische Stabilität des Gesamtsystems gewährleistet ist. Gemäß der Erfindung wird diese Aufgabe durch die Merkmale des kennzeichnenden Teils des Patentanspruchs 1 gelöst.

Mit der Erfindung ist ein Körper mit einer verbesserten Oberflächenschicht, insbesondere ein medizinisches Implantat oder eine medizinische Prothese geschaffen, bei welcher die besonderen Vorteile von Zirkonoxiden für biologische Anwendungen genutzt werden und bei der gleichzeitig eine hohe mechanische Stabilität des Gesamtsystems gewährleistet ist. Die Oberfläche ist aus einem mikroporösen Schichtoxidwerkstoff auf Zirkoniumoxidbasis ausgebildet und der Grundkörper besteht aus einem Basismetall, insbesondere aus Titan oder Titanlegierungen. Dabei ist die Oberfläche fest mit dem Grundkörper verbunden und zeigt auf Grund Ihrer Kombination von Oberflächenstrukturen und chemischen Zusammensetzung eine hohe Attraktivität für biologische Integrationsprozesse.

Unter dem Begriff "Basismetall" werden folgende medizinische Metalle verstanden: Titan (Ti), Aluminium (AI), Tantal (Ta), Niob (Nb), Wolfram (W) sowie deren Legierungen.

In Weiterbildung der Erfindung weist die Schicht eine Zirkonoxid-Konzentration von 5 bis 90 Massenprozent, vorzugsweise 25 bis 80 Massenprozent auf. Es hat sich gezeigt, dass bei diesen Metalloxidanteilen die mechanische und chemische Stabilität der Schicht maximiert ist.

Bevorzugt beträgt die Dicke der Schicht von 0,2 bis 40 µm, bevorzugt von 1,2 bis 7,5 µm. Eine solche Schichtdicke ist vorteilhaft bezüglich der Adhäsion und der Wahrung der Geometrie des Grundkörpers nach der Beschichtung.

In Ausgestaltung der Erfindung weist die Porenstruktur der Schicht geschlossene Poren auf, wobei die geschlossenen Poren einen maximalen Durchmesser von 1 µm aufweisen. Diese Poren verhindern bei einer möglichen Beschädigung der Schicht die Weiterleitung von Rissen. Bevorzugt weist die Porenstruktur der Schicht offene Poren auf, wobei die offenen Poren einen maximalen Durchmesser von 5 µm aufweisen und die Eindringtiefe der offenen Poren in der Schicht maximal das Dreifache ihres Durchmessers beträgt. Vorteilhaft weist die Porenstruktur der Schicht zylinderförmige Kanalporen auf, wobei die Länge der zylinderförmigen Kanalporen mehr als das Dreifache ihres Durchmessers beträgt. Die Kanalporen können bis zu der Basismetalloxidgrundschicht reichen, ohne aber eine direkte Verbindung zum Basismaterial zu bilden.

Vorteilhaft besitzt die Oberfläche neben der Porenstruktur zusätzlich eine Mikrorauhigkeit in Form von kegelstumpfförmigen und halbkugelförmigen Oxidausbildungen auf der Oberfläche. Die Höhe dieser Oxidausbildungen ist abhängig von der Schichtdicke der Zirkonoxid basierten Schicht und liegt bei maximal 80 % der mittleren Schichtdicke. Dieses System von Poren und Mikrostrukturierung begünstigt besonders den initialen Osteoblastenkontakt mit der Ausbildung von Filiaepodia.

In weiterer Ausgestaltung der Erfindung enthält die Schicht maximal 1 % chemisch gebundenen Schwefel. Der Schwefel wirkt in der vorliegenden Form als nukleophiles Zentrum und fördert besonders die Absorbtion von biologisch aktiven Molekülen, welche wesentlich an Knochenbildungsmechanismen beteiligt sind.

Die erfindungsgemäße Oberflächenschicht zeichnet sich durch ihren Aufbau dadurch aus, dass im Grenzbereich Basismetallsubstrat / Oberflächenschicht die Schicht aus reinem Basismetalloxid besteht und somit eine optimale Schichthaftung am Grundmetall erreicht wird. Dieser Schichtabschnitt geht in eine Zirkonium/Basismetall-Mischoxidregion über, wobei der Anteil an Zirkonoxid zur Oberfläche der Schicht hin ansteigt. Die Beschichtung enthält amorphe als auch kristalline Phasen der am Schichtaufbau beteiligten Oxidverbindungen. Die kristalline Hauptphase der Beschichtung ist monoklines Zirkonoxid, die stabile Tieftemperaturform dieses Oxides. Die Schicht ist frei von den thermodynamisch instabilen tetragonalen und kubischen Hochtemperaturformen des Zirkonoxides. Bei der Oberflächenschicht handelt es sich somit um ein mehrphasiges Oxidschichtsystem, welches als Phasen zumindest monoklines Zirkonoxid, Zirkonoxid/Basismetall-Mischoxid und Basismetalloxid aufweist. Bevorzugt ist das Basismetall Titan. Alternativ zum Zirkonoxid kann die biokompatible Oberflächenschicht auch aus einer Aluminiumoxidschicht gebildet sein. Diese beiden Materialien sind besonders biokompatibel.

In bevorzugter Ausgestaltung der Erfindung ist auf der Oberflächenschicht zumindest partiell eine weitere Schicht aufgebracht. Hierdurch ist eine Farbkorrektur des im Wesentlichen gräulich erscheinenden Zirkonoxids ermöglicht. Die weitere Schicht ist bevorzugt zahnfarben und mundbeständig ausgebildet.

Der Erfindung liegt weiterhin die Aufgabe zu Grunde, ein Verfahren zur Beschichtung eines Substrats zu schaffen, welches die bekannten Nachteile des Standes der Technik vermeidet, bei dem die besonderen Vorteile von Zirkonoxiden für biologische Anwendungen genutzt werden und bei dem gleichzeitig eine hohe mechanische Stabilität des Gesamtsystems gewährleistet ist. Gemäß der Erfindung wird diese Aufgabe durch die Merkmale des Patentanspruchs 14 gelöst.

Mit der Erfindung ist ein Verfahren zur Beschichtung eines Substrats aus einem Basismetall geschaffen, welches die bekannten Nachteile des Standes der Technik vermeidet und eine verbesserte Oberflächenschicht insbesondere für medizinische Implantate und Prothesen ermöglicht, bei welcher die besonderen Vorteile von Zirkonoxiden für biologische Anwendungen genutzt werden und bei dem gleichzeitig eine hohe mechanische Stabilität des Gesamtsystems gewährleistet ist. Es hat sich überraschender Weise gezeigt, dass in einem Einstufenprozess eine poröse, zirkonoxidbasierte, haftfeste und gleichzeitig biokompatible Oberfläche auf Basismetallsubstraten, insbesondere Titansubstraten geschaffen werden kann. Die Vorbehandlung des zu beschichtenden Substrats erfolgt nach dem Stand der Technik, vorzugsweise durch eine Kombination von Säurebehandlung und Spülprozessen. Anschließend wird das gereinigte Beschichtungsgut als Anode in eine Elektrolysezelle positioniert. Überraschend hat sich gezeigt, dass bei Verwendung eines Zirkon(IV)sulfat-Elektrolyten die Bildung von Schichtoxiden mit dominierenden ZrO₂-Anteilen möglich ist. Bei bisher bekannten Verfahren der anodischen Konversionsschichtbildung bildet immer die mit dem zu beschichtenden Grundmetall korrespondierende Oxidphase das vorherrschende Schichtoxid. Die Herstellung der Schicht, die Ausbildung der Porosität und die Mikrostrukturierung der Oberfläche erfolgen simultan in einem Verfahrensschritt. Bevorzugt liegt die Konzentration des Basiselektrolyten im Bereich von 0,001 Mol/I bis 2,9 Mol/I Zirkon(IV)sulfat.

In weiteren bevorzugten Ausführungen der Erfindung können diesem Basiselektrolyt Fluoride des Li, Na oder K in Konzentrationen von 0,001 Mol/I bis 8,5 Mol/I und / oder Phosphorsäure in Konzentrationen von 0,001 Mol/I bis 3,3 Mol/I zugesetzt werden.

Der Schichtbildungsprozess erfolgt in drei Regimen: Zunächst wird die haftvermittelnde, stabile Basismetalloxidgrundschicht durch anodische Polarisation hergestellt. In einem bevorzugten Ausführungsbeispiel wird dabei potentiodynamisch das Anodenpotenzial erhöht, wobei die Stromdichte 0,01 bis 1,2 A/cm², vorzugsweise 0,1 bis 0,9 A/cm² nicht überschreiten darf. Anschließend wird das Anodenpotenzial weiter erhöht, so dass in einem quasi potentiostatischen Regime der Anodenstrom zwischen 0,01 A/cm² und 3,2 A/cm² zur Beschichtung genutzt wird. Abschließend erfolgt eine Konditionierung der Schicht, indem die Zellspannung reduziert wird und für einen Zeitraum von 0,1 Min. bis 10 Min., vorzugsweise 0,5 Min. bis 2 Min. eine Endpassierung bei Anodenströmen von kleiner 0,5 A/cm² erfolgt. Die Gesamtzeit des Schichtbildungsprozesses beträgt zwischen 1 Min. und 50 Min., vorzugsweise zwischen 2 Min. und 8 Min.

Andere Weiterbildungen und Ausgestaltungen sind in den übrigen Unteransprüchen angegeben. An Hand von zwei Ausführungsbeispielen wir nachfolgend die Erfindung im Einzelnen beschrieben.

### Ausführungsbeispiel 1:

Ein Zylinder aus Reintitan mit den Abmessungen Höhe = 12 mm und Durchmesser = 4 mm wird für 120 Sekunden in einem Ultraschallbad mit Isopropanol gereinigt und anschließend getrocknet. Als weitere Vorbehandlung erfolgt eine Beizbehandlung für 15 Sekunden in einer Lösung, welche zu 25 % aus konzentriertem HNO₃, zu 25 % aus konzentriertem HF und zu 50 % aus konzentriertem H₃PO₄ besteht. Anschließend erfolgt ein mehrfaches Spülen im destillierten Wasser und eine Endreinigung in Isopropanol.

Nach dem Trocknen wird die Titanprobe in einer elektrolytischen Zelle als Anode positioniert. Der verwendete Elektrolyt besteht aus einer wässrigen Lösung (25°C) von 0,124 Mol/I Zirkonium (IV)-Sulfat.

Das Regime der anodischen Behandlung ist wie folgt: 60 Sekunden erfolgt eine potentiodynamische Anodisierung, wobei die Stromdichte 0,1 A/cm² nicht überschreiten darf. Anschließend wird das Anodenpotenzial soweit erhöht, dass der Anodenstrom für 120 Sekunden im Bereich zwischen 0,3 und 0,9 A/cm² liegt. Es erfolgt eine Endpassivierung durch Reduktion des Anodenpotenzials, so dass für 50 Sekunden der Anodenstrom 0,06 A/cm² nicht übersteigt. Anschließend wird die Probe aus der Anodisierzelle entfernt und mit Wasser und Isopropanol gespült und getrocknet.

Es entsteht eine stark strukturierte, poröse Oberfläche, mit Porendurchmessern bis zu 2 Mikrometern, wobei die Distanz zwischen den einzelnen Poren die gleiche Größenordnung aufweist. Die Oberfläche enthält in diesem Ausführungsbeispiel 80 % ZrO₂. Der mittlere Gehalt der Schicht an Schwefel beträgt 0,1 %.

### Ausführungsbeispiel 2:

Ein Zylinder mit Außengewinde aus Reintitan mit den Abmessungen Höhe = 11 mm und Durchmesser = 3 mm wird für 120 Sekunden in einem Ultraschallbad mit Isopropanol gereinigt und anschließend getrocknet. Als weitere Vorbehandlung erfolgt eine Beizbehandlung für 15 Sekunden in einer Lösung, welche zu 25 % aus konzentriertem HNO₃, zu 25 % aus konzentriertem HF und zu 50 % aus konzentriertem H₃PO₄ besteht. Anschließend erfolgt ein mehrfaches Spülen im destillierten Wasser und eine Endreinigung in Isopropanol.

Nach dem Trocknen wird die Titanprobe in einer elektrolytischen Zelle als Anode positioniert. Der verwendete Elektrolyt setzt sich aus einer wässrigen Lösung (25°C) von 44 g/l Zirkonium(IV)sulfat, 0,3 I einer 15 % KF-Lösung und 0,015 I konzentrierter Phosphorsäure zusammen, das vorliegende Gesamtvolumen wird anschließend im Verhältnis 1:1 1 mit Wasser verdünnt.

Das Regime der anodischen Behandlung ist wie folgt: 80 Sekunden erfolgt eine potentiodynamische Anodisierung, wobei die Stromdichte 0,1 A/cm² nicht überschreiten darf. Anschließend wird das Anodenpotenzial soweit erhöht, dass der Anodenstrom für 230 Sekunden im Bereich zwischen 0,2 und 0,8 A/cm² liegt. Es erfolgt eine Endpassivierung durch Reduktion des Anodenpotenzials, so dass für 60 Sekunden der Anodenstrom 0,05 A/cm² nicht übersteigt. Die Probe wird anschließend aus der Anodisierzelle entfernt und mit Wasser und Isopropanol gespült und getrocknet.

Es entsteht eine stark strukturierte, poröse Oberfläche, mit Porendurchmessern bis zu 3,5 Mikrometern. Die Oberfläche enthält in diesem Ausführungsbeispiel 47 % ZrO₂.

## Patentansprüche

1. Körper, insbesondere medizinisches Implantat oder medizinische Prothese, bestehend aus einem Basismetallgrundkörper mit einer biokompatiblen Oberflächenschicht, die aus Zirkonoxid sowie einem Basismetalloxid gebildet ist, **dadurch gekennzeichnet, dass** die Schicht als mikroporöser Schichtoxidwerkstoff ausgebildet ist und entlang ihres Querschnitts in Richtung ihrer äußeren Oberfläche einen zunehmenden Zirkonoxidanteil sowie einen abnehmenden Basismetalloxidanteil aufweist, wobei das Basismetall eines der folgenden medizinischen Metalle ist: Titan (Ti), Aluminium (Al), Tantal (Ta), Niob (Nb), Wolfram (W) sowie deren Legierungen und wobei im Grenzbereich Basismetall / Oberflächenschicht die Oberflächenschicht aus reinem Basismetalloxid besteht.

2. Körper nach Anspruch 1, **dadurch gekennzeichnet, dass** die Schicht eine Zirkonoxid -Konzentration von 5 bis 90 Massenprozent, vorzugsweise 25 bis 80 Massenprozent aufweist.

3. Körper nach Anspruch 1 oder 2, **dadurch gekennzeichnet, dass** die Dicke der Schicht von 0,2 bis 40 µm, bevorzugt 1,2 bis 7,5 µm beträgt.

4. Körper nach einem der vorgenannten Ansprüche, **dadurch gekennzeichnet, dass** die Porenstruktur der Schicht geschlossene Poren aufweist, wobei die geschlossenen Poren einen maximalen Durchmesser von 1 µm aufweisen.

5. Körper nach einem der vorgenannten Ansprüche, **dadurch gekennzeichnet, dass** die Porenstruktur der Schicht offenen Poren aufweist, wobei die offenen Poren einen maximalen Durchmesser von 5 µm aufweisen und die Eindringtiefe der offenen Poren in die Schicht maximal das Dreifache ihres Durchmessers beträgt.

6. Körper nach einem der vorgenannten Ansprüche, **dadurch gekennzeichnet, dass** die Porenstruktur der Schicht zylinderförmigen Kanalporen aufweist, wobei die Länge der zylinderförmigen Kanalporen mehr als das Dreifache ihres Durchmessers beträgt.

7. Körper nach einem der vorgenannten Ansprüche, **dadurch gekennzeichnet, dass** die Schicht maximal ein Prozent chemisch gebundenen Schwefel enthält.

8. Körper nach einem der vorgenannten Ansprüche, **dadurch gekennzeichnet, dass** die kristalline Hauptphase der Beschichtung monoklines Zirkonoxid ist.

9. Körper nach einem der vorgenannten Ansprüche, **dadurch gekennzeichnet, dass** die Schicht keine tetragonalen und kubischen Hochtemperaturformen des Zirkonoxides enthält.

10. Körper nach einem der vorgenannten Ansprüche, **dadurch gekennzeichnet, dass** auf der Oberflächenschicht zumindest partiell eine weitere Schicht aufgebracht ist.

11. Körper nach Anspruch 10, **dadurch gekennzeichnet, dass** die weitere Schicht zahnfarben und mundbeständig ausgebildet ist.

12. Körper nach einem der vorgenannten Ansprüche, **dadurch gekennzeichnet, dass** der Grundkörper ein zahnmedizinisches Implantat oder ein Element eines Aufbaus für ein zahnmedizinisches Implantat ist.

13. Körper nach einem der vorgenannten Ansprüche, **dadurch gekennzeichnet, dass** das Basismetall Titan ist.

14. Verfahren zur Beschichtung eines Substrats aus einem Basismetall zur Erzielung einer mikroporösen Oberflächenschicht, wobei das Substrat zunächst durch eine Kombination von Säurebehandlung und Spülprozessen vorbehandelt wird und anschließend als Anode in einer Elektrolysezelle positioniert wird, **dadurch gekennzeichnet, dass** die Beschichtung in einem wässrigen 0,001 mol/l bis 2,9 mol/l Zirkon(IV)sulfat enthaltenen Elektrolyt erfolgt.

15. Verfahren nach Anspruch 14, **dadurch gekennzeichnet, dass** dem Elektrolyt wenigstens ein Fluorid des Li, Na oder K in einer Konzentration von 0,001 mo/l bis 8,5 mol/l zugesetzt wird.

16. Verfahren nach Anspruch 14 oder 15, **dadurch gekennzeichnet, dass** dem Elektrolyt Phosphorsäure in einer Konzentration von 0,001 mol/l bis 3,3 mol/l zugesetzt wird.

17. Verfahren nach einem der Ansprüche 14 bis 16, **dadurch gekennzeichnet, dass** zuerst eine haftvermittelnde, stabile Metalloxidgrundschicht durch anodische potentiodynamische Polarisation hergestellt wird, anschließend das Anodenpotential weiter erhöht wird, sodass in einem quasipotentiostatischen Regime der Anodenstrom zwischen 0,01 A/cm² und 3 A/cm² zur Beschichtung genutzt wird und abschließend eine Konditionierung der Schicht erfolgt, indem die Zellspannung reduziert wird und für einen Zeitraum von 0,1 Minuten und 10 Minuten eine Endpassivierung bei Anodenströmen von kleiner 0,5 A/cm² erfolgt.

18. Verfahren nach einem der Ansprüche 14 bis 17, **dadurch gekennzeichnet, dass** das Basismetall Titan ist.

## Claims

1. Body, in particular a medical implant or medical prosthesis, consisting of a basic body of base metal with a bio-compatible surface layer formed of zirconium oxide and a base-metal oxide, **characterised in that** the layer is formed as a microporous stratified-oxide material and has an increasing content of zirconium oxide and a decreasing content of base-metal oxide along its cross-section towards its outer surface, where the base metal is one of the following medical metals: titanium (Ti), aluminium (Al), tantalum (Ta), niobium (Nb), tungsten (W) and their alloys, and where the surface layer is of pure base-metal oxide in the area of transition from the base metal to the surface layer.

2. Body in accordance with claim 1, **characterised in that** the layer has a concentration of zirconium oxide of between 5 and 90 percent weight by weight, preferably between 25 and 80 percent weight by weight.

3. Body in accordance with claim 1 or claim 2, **characterised in that** the thickness of the layer is between 0,2 and 40 µm, and preferably between 1,2 and 7,5 µm.

4. Body in accordance with any of the aforementioned claims, **characterised in that** the pore structure of the layer has closed pores, and where the closed pores have a maximum diameter of 1 µm.

5. Body in accordance with any of the aforementioned claims, **characterised in that** the pore structure of the layer has open pores, and where the open pores have a maximum diameter of 5 µm and the depth of penetration of the open pores into the layer is, at the most, three times their diameter.

6. Body in accordance with any of the aforementioned claims, **characterised in that** the pore structure of the layer has cylinder-shaped channel pores, where the length of the cylinder-shaped channel pores is more than three times their diameter.

7. Body in accordance with any of the aforementioned claims, **characterised in that** the layer contains a maximum of one percent of chemically bonded sulphur.

8. Body in accordance with any of the aforementioned claims, **characterised in that** the main crystalline phase of the layer is monoclinic zirconium oxide.

9. Body in accordance with any of the aforementioned claims, **characterised in that** the layer contains no tetragonal or cubic high-temperature forms of zirconium oxide.

10. Body in accordance with any of the aforementioned claims, **characterised in that** another layer is applied, at least partially, to the surface layer.

11. Body in accordance with claim 10, **characterised in that** the other layer is tooth-coloured and stable under oral conditions.

12. Body in accordance with any of the aforementioned claims, **characterised in that** the basic body is a dental implant or an element for the construction of a dental implant.

13. Body in accordance with any of the aforementioned claims, **characterised in that** the base metal is titanium.

14. Process for coating a base-metal substrate in order to achieve a microporous surface layer, where the substrate is first of all subjected to a combination of acid treatment and rinsing processes and is then positioned as the anode in an electrolytic cell, **characterised in that** the coating process takes place in an aqueous electrolyte containing between 0,001 mol/I and 2,9 mol/I of zirconium (IV) sulphate.

15. Process in accordance with claim 14, **characterised in that** at least one fluoride of Li, Na or K is added to the electrolyte in a concentration of between 0,001 mol/I and 8,5 mol/l.

16. Process in accordance with claim 14 or claim 15, **characterised in that** phosphoric acid is added to the electrolyte in a concentration of between 0,001 mol/I and 3,3 mol/l.

17. Process in accordance with any of the claims 14 to 15, **characterised in that**, first of all, a stable grounding layer of metallic oxide is formed as a bonding agent by means of anodic potentiodynamic polarisation, the anode potential is then further increased so that, in a quasi potentiostatic system, the anode current between 0,01 A/cm² und 3 A/cm² is used for coating, and finally, the layer is conditioned by reducing the cell voltage and carrying out a final passivation operation at anode currents of less than 0,5 A/cm² over a period of between 0,1 and 10 minutes.

18. Process in accordance with any of the claims 14 to 17, **characterised in that** the base metal is titanium.

## Revendications

1. Structure, implant médical ou prothèse médicale notamment, comprenant une structure principale élaborée dans un métal de base recouverte d'une couche superficielle biocompatible, laquelle couche est constituée d'oxyde de zirconium et d'oxyde de métal de base, **caractérisée en ce que** la couche comprend un matériau de couche microporeux à base d'oxyde et que ladite couche présente vers sa face extérieure le long de sa section transversale une part croissante d'oxyde de zirconium et une part décroissante d'oxyde de métal de base, lequel métal de base est l'un des métaux médicaux suivants: titane (Ti), aluminium (Al), tantale (Ta), niobium (Nb), tungstène (W) et leurs alliages, laquelle couche superficielle est constituée, dans la zone limite métal de base / couche superficielle, d'oxyde pur de métal de base.

2. Structure selon la revendication 1 **caractérisée en ce que** la couche présente une concentration d'oxyde de zirconium de 5 à 90 % en poids, de préférence de 25 à 80 % en poids.

3. Structure selon la revendication 1 ou 2 **caractérisée en ce que** l'épaisseur de la couche atteint 0,2 à 40 µm, de préférence 1,2 à 7,5 µm.

4. Structure selon l'une des revendications précédentes **caractérisée en ce que** la structure poreuse de la couche présente des pores fermés, lesdits pores fermés présentant un diamètre maximum de 1 µm.

5. Structure selon l'une des revendications précédentes **caractérisée en ce que** la structure poreuse de la couche présente des pores ouverts, lesdits pores ouverts présentant un diamètre maximum de 5 µm et dont la profondeur de pénétration dans la couche atteint au maximum trois fois leur diamètre.

6. Structure selon l'une des revendications précédentes **caractérisée en ce que** la structure poreuse de la couche présente des pores en canal cylindriques, dont la longueur dépasse trois fois leur diamètre.

7. Structure selon l'une des revendications précédentes **caractérisée en ce que** la couche comprend au maximum 1 % de soufre chimiquement lié.

8. Structure selon l'une des revendications précédentes **caractérisée en ce que** la phase principale cristalline du revêtement est constituée d'oxyde de zirconium monoclinique.

9. Structure selon l'une des revendications précédentes **caractérisée en ce que** la couche ne comprend pas de formes d'oxyde de zirconium tétragonales et cubiques obtenues à haute température.

10. Structure selon l'une des revendications précédentes **caractérisée en ce qu'**au moins en partie une autre couche est appliquée sur la couche superficielle.

11. Structure selon la revendication 10 **caractérisée en ce que** l'autre couche est façonnée dans la teinte des dents et résistante à l'oxydation.

12. Structure selon l'une des revendications précédentes **caractérisée en ce que** la structure principale est un implant médico-dentaire ou un élément de structure pour implant médico-dentaire.

13. Structure selon l'une des revendications précédentes **caractérisée en ce que** le métal de base est le titane.

14. Procédé destiné à revêtir un support élaboré dans un métal de base en vue d'obtenir une couche superficielle microporeuse, lequel support subit tout d'abord un traitement préalable par association d'un traitement à l'acide et de phases de rinçage pour être ensuite mis en oeuvre comme anode dans une cellule électrolytique, **caractérisé en ce que** le revêtement est obtenu dans un électrolyte aqueux de 0,001 mol/l à 2,9 mol/l de sulfate de zirconium (IV).

15. Procédé selon la revendication 14 **caractérisé en ce qu'**au moins un fluorure de Li, Na ou K est ajouté à l'électrolyte en concentration de 0,001 mol/l à 8,5 mol/l.

16. Procédé selon l'une des revendications 14 ou 15 **caractérisé en ce qu'**un acide phosphorique est ajouté à l'électrolyte en concentration de 0,001 mol/l à 3,3 mol/l.

17. Procédé selon l'une des revendications 14 à 16 **caractérisé en ce que** tout d'abord une couche principale en oxyde métallique stable et favorisant l'adhérence est élaborée par polarisation potentiodynamique anodique, qu'ensuite, pour le revêtement, le potentiel anodique est encore augmenté au point que le courant anodique varie entre 0,01 A/cm² et 3 A/cm² en régime pour ainsi dire potentiostatique, et qu'enfin la couche est conditionnée en réduisant la tension de pile et en produisant dans un laps de temps de 0,1 min à 10 min une passivation terminale aux courants anodiques inférieurs à 0,5 A/cm².

18. Procédé selon l'une des revendications 14 à 17 **caractérisé en ce que** le métal de base est le titane.
